# EUROPEAN PATENT APPLICATION

(11) **EP 0 982 318 A2**
(43) Date of publication of application: **01.03.2000**
(21) Application number: 99306775.0
(22) Date of filing: 26.08.1999
(51) Int. Cl.: C07K 16/40, G01N 33/573, C12Q 1/48

(54) **Enchanced phosphorylation of P53 by ATM in response to DNA damage**

(30) Priority: 26.08.1998 US 97898 P
(71) Applicant: RAMOT UNIVERSITY AUTHORITY FOR APPLIED RESEARCH & INDUSTRIAL DEVELOPMENT LTD., Tel Aviv 61392 (IL)
(72) Inventor: Shiloh, Yosef, Ramat Aviv, Tel Aviv (IL); Smorodinsky, Nehama I., Hertzeila Pituach (IL)
(74) Representative: Gallafent, Antony Xavier

(57) **Abstract**

There are provided antibodies directed against the ATM protein are peptide derivatives of the protein are also provided. Also provided is a diagnostic tool for determining the presence of A-T having a detector for detecting ATM protein levels and quantification tools for analyzing the ATM protein levels. A kit for detecting the presence of A-T containing a detector for detecting ATM protein levels and a quantificator for analyzing ATM protein levels is also provided.

## Description

The present invention relates to the field of gene therapy. More specifically, the present invention relates to a method of improving DNA stability in response to damage inducing events in both normal and genetically compromised cells.

Strand breaks in cellular DNA occur continuously as a consequence of normal processes such as recombination or the infliction of DNA damage. DNA damage triggers several signal transduction pathways that lead either to damage repair coupled with attenuation of cell cycle progression, or to programmed cell death (apoptosis). A junction of such pathways is controlled by the transcription factor p53. After DNA damage, the amount of p53 in cells is increased through post-transcriptional mechanisms and its transactivation activity is enhanced, leading to the activation of downstream genes [ref 1]. The resultant activation of downstream genes may lead to NA repair and temporary cell cycle arrest, or to apoptosis.

Genetic disorders disrupting such pathways usually result in genomic instability, degeneration of specific tissues, hypersensitivity to particular DNA damaging agents, and cancer predisposition. One of these "genome instability syndromes" is ataxia-telangiectasia (A-T), characterized by cerebellar and thymic degeneration, immunodeficiency, gonadal dysgenesis, hypersensitivity to ionizing radiation, and extreme predisposition to lymphoreticular malignancies. Cultured cells from A-T patients or ATM-deficient mice exhibit reduced life span, chromosomal instability, acute sensitivity to ionizing radiation and radiomimetic chemicals, and failure to activate cell cycle checkpoints following treatment with these agents. Typically, ionizing radiation-induced accumulation of p53 and the subsequent activation of downstream genes are delayed when compared to normal cells.

The responsible gene, ATM, encodes a 370 kDa protein with a carboxy-terminal domain similar to the catalytic subunit of phosphatidylinositol 3-kinases (P1 3-kinases). This similarity places ATM within a family of large proteins sharing the PI 3-kinase related domain, which were identified in different organisms and found to be involved in maintaining mitotic and meiotic stability, in cell cycle control, and in cellular responses to DNA damage. These proteins are generally considered protein kinases rather than lipid kinases. The most extensively characterized member of this family is the DNA-dependent protein kinase (DNA-PK), which is involved in processing DNA double-strand breaks and probably in signaling their presence via phosphorylation of other proteins. DNA-PK phosphorylates *in vitro* numerous proteins, including p53. Another family member shown unequivocally to be a protein kinase is mTOR (also designated FRAP and RAFT1), which is involved in regulating cell cycle progression through the G1 phase.

It would therefore be useful to develop methods and compositions which could improve DNA stability in response to damage inducing events.

According to the present invention, there are provided, antibodies directed against the ATM protein and peptide derivatives of the protein.

Also provided is a diagnostic tool for determining the presence and activity of ATM the diagnostic tool containing a detector for detecting ATM protein levels and quantificator for analyzing the ATM protein levels. A kit for detecting the presence and activity of ATM containing a detector for detecting ATM protein levels and a quantificator for analyzing ATM protein levels is also provided.

Other advantages of the present invention will be readily appreciated as the same becomes better understood by reference to the following detailed description when considered in connection with the accompanying drawings wherein:
Figures 1 A-D show the kinase activity of ATM with PHAS-I as a substrate;
Figures 2 A-D show the effects of phosphorylation of Ser15 on p53;
Figures 3 A and B show the increase kinase activity of ATM precipitates from cells treated with NCS.

Generally, the present invention relates to antibodies directed against the protein or peptide derivatives produced as described herein in the methods and Example. These antibodies can be used in determining the presence of the ATM protein. Specifically, these antibodies recognize a 370 kD protein in normal cells which is not detected in A-T cells but reappears upon ectopic expression of ATM in them. These antibodies also recognize endogenous, recombinant and in-vitro translated ATM immunoprecipitated by other anti-ATM antibodies.

The ATM protein, encoded by the gene responsible for the human genetic disorder ataxia-telangiectasia (AT), regulates several cellular responses to DNA breaks. ATM shares a phosphoinositide 3-kinase-related domain with several proteins, some of them protein kinases. A wortmannin-sensitive protein kinase activity was associated with endogenous or recombinant ATM, and was abolished by AT mutations*. In vitro* substrates included the translation repressor PHAS-I and the p53 protein. ATM phosphorylated p53 *in vitro* on a single residue, Ser15, which is phosphorylated *in vivo* in response to DNA damage. This activity was enhanced several-fold minutes after treatment of cells with a radiomimetic drug; total amount of ATM remained unchanged. Various damage-induced responses are activated by enhancement of the protein kinase activity of ATM. Therefore therapeutic measures to enhance this response to damage activity are needed in those cases where DNA damage is occurring and normal cell regulation is not adequate to respond.

For producing polyclonal antibodies a host, such as a rabbit or goat, is immunized with the immunogen or immunogen fragment, generally with an adjuvant and, if necessary, coupled to a carrier; antibodies to the immunogen are collected from the sera. Further, the polyclonal antibody can be absorbed such that it is monospecific. That is, the sera can be absorbed against related immunogens so that no cross-reactive antibodies remain in the sera rendering it monospecific.

For producing monoclonal antibodies the technique involves hyperimmunization of an appropriate donor with the immunogen, generally a mouse, and isolation of splenic antibody producing cells. These cells are fused to a cell having immortality, such as a myeloma cell, to provide a fused cell hybrid which has immortality and secretes the required antibody. The cells are then cultured, in bulk, and the monoclonal antibodies harvested from the culture media for use.

The present invention further provides a method of studying ATM activity by raising antibodies against ATM-derived peptides, immunoprecipitating ATM proteins from the cultured cells, and assaying the immunoprecipitates. This method enables one of skill in the art to observe that the activity of ATM is intrinsic protein kinase activity. More specifically, endogenous or recombinant ATM has an associated wortmannin-sensitive protein kinase activity which is abolished by AT mutations.

The present invention also provides a diagnostic tool for determining the presence and activity of ATM containing detection means for detecting ATM protein levels and quantification means for analyzing the ATM protein activity.

There is also provided a kit for detecting the presence and activity of ATM having therein detection means for detecting ATM protein levels and quantification means for analyzing the ATM protein activity.

The above discussion provides a factual basis for the use of compositions for modulating the ATM protein. The methods used with the utility of the present invention can be shown by the following non-limiting examples and accompanying figures.

### EXAMPLES

### GENERAL METHODS:

General methods in molecular biology: Standard molecular biology techniques known in the art and not specifically described are generally followed as in Sambrook et al., *Molecular Cloning: A Laboratory Manual*, Cold Springs Harbor Laboratory, New York (1989, 1992), and in Ausubel et al., *Current Protocols in Molecular Biology*, John Wiley and Sons, Baltimore, Maryland (1989). Polymerase chain reaction (PCR) is carried out generally as in *PCR Protocols: A Guide To Methods And Applications*, Academic Press, San Diego, CA (1990). Reactions and manipulations involving other nucleic acid techniques, unless stated otherwise, are performed as generally described in Sambrook et al., 1989, *Molecular Cloning: A Laboratory Manual*, Cold Spring Harbor Laboratory Press, and methodology as set forth in United States patents 4,666,828; 4,683,202; 4,801,531; 5,192,659 and 5,272,057 and incorporated herein by reference. *In-situ* (In-cell) PCR in combination with Flow Cytometry can be used for detection of cells containing specific DNA and mRNA sequences (Testoni et al, 1996, Blood 87:3822.)

General methods in immunology: Standard methods in immunology known in the art and not specifically described are generally followed as in Stites et al.(eds), Basic and Clinical Immunology (8th Edition), Appleton & Lange, Norwalk, CT (1994) and Mishell and Shiigi (eds), Selected Methods in Cellular Immunology, W.H. Freeman and Co., New York (1980).

### Immunoassays

In general, ELISAs are one immunoassay employed to assess a specimen. ELISA assays are well known to those skilled in the art. Both polyclonal and monoclonal antibodies can be used in the assays. Where appropriate other immunoassays, such as radioimmunoassays (RIA) can be used as are known to those in the art. Available immunoassays are extensively described in the patent and scientific literature. See, for example, United States patentS 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521 as well as Sambrook et al, *Molecular Cloning: A Laboratory Manual*, Cold Springs Harbor, New York, 1989.

### Antibody Production

Antibody Production: Antibodies may be either monoclonal, polyclonal or recombinant and are further described in the Example herein. Conveniently, the antibodies may be prepared against the immunogen or portion thereof for example a synthetic peptide based on the sequence, or prepared recombinantly by cloning techniques or the natural gene product and/or portions thereof may be isolated and used as the immunogen. Immunogens can be used to produce antibodies by standard antibody production technology well known to those skilled in the art as described generally in Harlow and Lane, *Antibodies: A Laboratory Manual*, Cold Spring Harbor Laboratory, Cold Spring Harbor, NY, 1988 and Borrebaeck, *Antibody Engineering - A Practical Guide*, W.H. Freeman and Co., 1992. Antibody fragments may also be prepared from the antibodies and include Fab, F(ab')₂, and Fv by methods known to those skilled in the art.

For producing polyclonal antibodies a host, such as a rabbit or goat, is immunized with the immunogen or immunogen fragment, generally with an adjuvant and, if necessary, coupled to a carrier; antibodies to the immunogen are collected from the sera. Further, the polyclonal antibody can be absorbed such that it is monospecific. That is, the sera can be absorbed against related immunogens so that no cross-reactive antibodies remain in the sera rendering it monospecific.

For producing monoclonal antibodies the technique involves hyperimmunization of an appropriate donor with the immunogen, generally a mouse, and isolation of splenic antibody producing cells. These cells are fused to a cell having immortality, such as a myeloma cell, to provide a fused cell hybrid which has immortality and secretes the required antibody. The cells are then cultured, in bulk, and the monoclonal antibodies harvested from the culture media for use.

For producing recombinant antibody (see generally Huston et al, 1991; Johnson and Bird, 1991; Mernaugh and Mernaugh, 1995), messenger RNAs from antibody producing B-lymphocytes of animals, or hybridoma are reverse-transcribed to obtain complimentary DNAs (cDNAs). Antibody cDNA, which can be full or partial length, is amplified and cloned into a phage or a plasmid. The cDNA can be a partial length of heavy and light chain cDNA, separated or connected by a linker. The antibody, or antibody fragment, is expressed using a suitable expression system to obtain recombinant antibody. Antibody cDNA can also be obtained by screening pertinent expression libraries.

The antibody can be bound to a solid support substrate or conjugated with a detectable moiety or be both bound and conjugated as is well known in the art. (For a general discussion of conjugation of fluorescent or enzymatic moieties see Johnstone & Thorpe, *Immunochemistry in Practice*, Blackwell Scientific Publications, Oxford, 1982.) The binding of antibodies to a solid support substrate is also well known in the art. (see for a general discussion Harlow & Lane *Antibodies: A Laboratory Manual*, Cold Spring Harbor Laboratory Publications, New York, 1988 and Borrebaeck, *Antibody Engineering - A Practical Guide*, W.H. Freeman and Co., 1992) The detectable moieties contemplated with the present invention can include, but are not limited to, fluorescent, metallic, enzymatic and radioactive markers such as biotin, gold, ferritin, alkaline phosphatase, β-galactosidase, peroxidase, urease, fluorescein, rhodamine, tritium, ¹⁴C and iodination.

### EXAMPLE 1

### METHOD:

For these experiments, 5X10⁶ cells were washed in ice-cold phosphate buffered saline and lysed in 700 µl of 10 mM Tris-HCl (pH 7.5), 150 mM NaCl, 0.2% dodecyl maltoside (Sigma), 5 mM EDTA, 50 mM NaF, 2 mM phenylmethylsulfonylfluoride, 100 µM NaVO4, 2 µg/ml aprotonin (lysis buffer). Freshly prepared cell lysates were incubated for 2 hours at 4°C on a rotator with 10 µl of hybridoma supernatants. Fifty µl of magnetic beads coated with anti-mouse IgG (Dynabeads M-450, DYNAL, Oslo, Norway) were added and the mixture was further rotated at 4°C for 2 hours. Immune complexes were collected using a magnetic stand (DYNAL) and washed 3 times with lysis buffer. For Western blotting analysis, 1/3 of this mixture was loaded on a polyacrylamide-SDS gel which was later blotted onto a nitrocellulose membrane (Nitrocell MFF, Pharmacia Biotech, San Francisco, CA) as previously described [ref 8]. Immune complexes were washed twice with kinase buffer (50 mM Hepes, pH 7.5, 150 mM NaCl, 4 mM MnCl2, 6 mM MgCl₂, 10% v/v glycerol, 1 mM DTT, 100 µM NaVO4). Two hundred ng - 1 µg substrate, 10 µCi [32P]ATP and ATP to final concentration of 5 µM were added, and the reaction mixtures were incubated at 30°C for 3-15 minutes. Following electrophoresis in SDS-polyacrylamide gels, the reaction products were visualized by autoradiography.

### Results

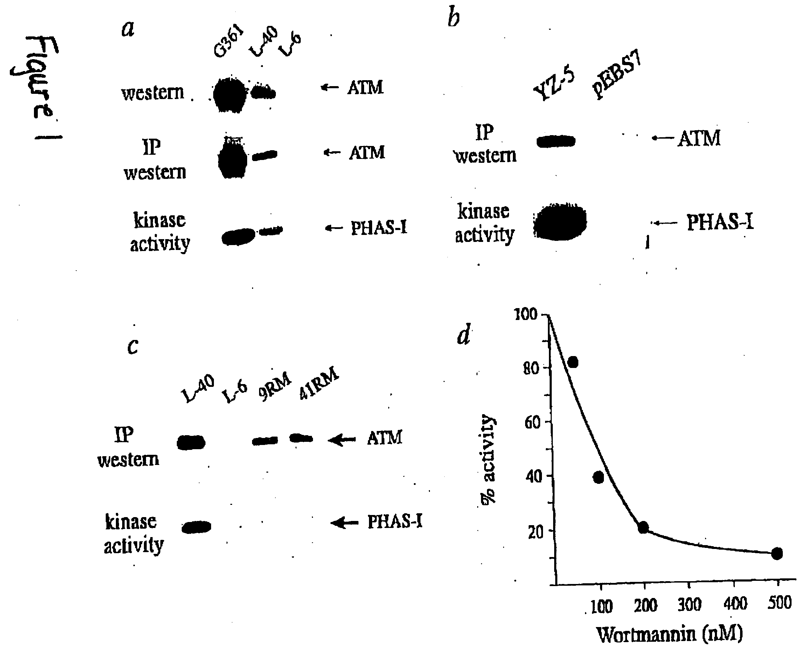

In order to study ATM's catalytic activity, monoclonal antibodies were raised to two ATM-derived peptides, ATM132 (spanning ATM positions 819-844) and ATML2 (positions 2581-2599). Both antibodies recognize a 370 kD protein in normal cells which is not detected in A-T cells but reappears upon ectopic expression of ATM in them. These antibodies also recognize endogenous, recombinant and in-vitro translated ATM immunoprecipitated by other anti-ATM antibodies. Protein kinase activity was revealed in immunoprecipitates obtained with these antibodies from a normal lymphoblastoid cell line (L-40), when the translation repressor PHAS-I [ref 9] was used as a substrate (Figure 1A). This activity was not detected with an unrelated antibody, and was retained when ATM was eluted from the beads using an excess of the peptide to which the antibody was raised. Phosphorylation of PHAS-I was considerably more intense with immunoprecipitates obtained from G361, a melanoma cell line in which the amount of ATM is greater than that in lymphoblasts (Figure 1A); it was not observed in immunoprecipitates obtained from an A-T cell line, L-6, that lacks the ATM protein (Figure 1A). Immunoprecipitates containing recombinant ATM expressed in A-T cells [ref 8] showed kinase activity similar to that of the endogenous protein (Figure 1B). Unlike DNA-PK, this activity was strictly dependent on manganese ions and was not enhanced by the addition of 200 ug of sheared double-stranded DNA, which considerably stimulated DNA-PK activity.

ATM-associated kinase activity was also measured in two A-T cell lines containing structural ATM mutations that allow expression of the mutant proteins. The cell line 41RM is homozygous for a missense mutation, E2904G [ref 11], that replaces a highly conserved glutamic acid residue by glycine within the PI 3-kinase-related domain that is expected to contain the catalytic site of the kinase. The cell line 9RM is homozygous for a nonsense mutation, R3047X, that removes the ten COOH-terminal residues of ATM (12). Little or no kinase activity was detected in immunoprecipitates from either cell line (Figure 1C). These results indicate that ATM has an intrinsic protein kinase activity.

PI 3-kinases are inhibited by wortmannin, which covalently modifies conserved lysine residues at their catalytic sites [ref 13]. ATM-associated kinase activity was inhibited in immune complexes exposed to wortmannin for 30 minutes (Figure 1D). The sensitivity of ATM to this drug (IC₅₀ = 100 nM) is comparable to that of other PI 3-kinase-related protein kinases [ref 5].

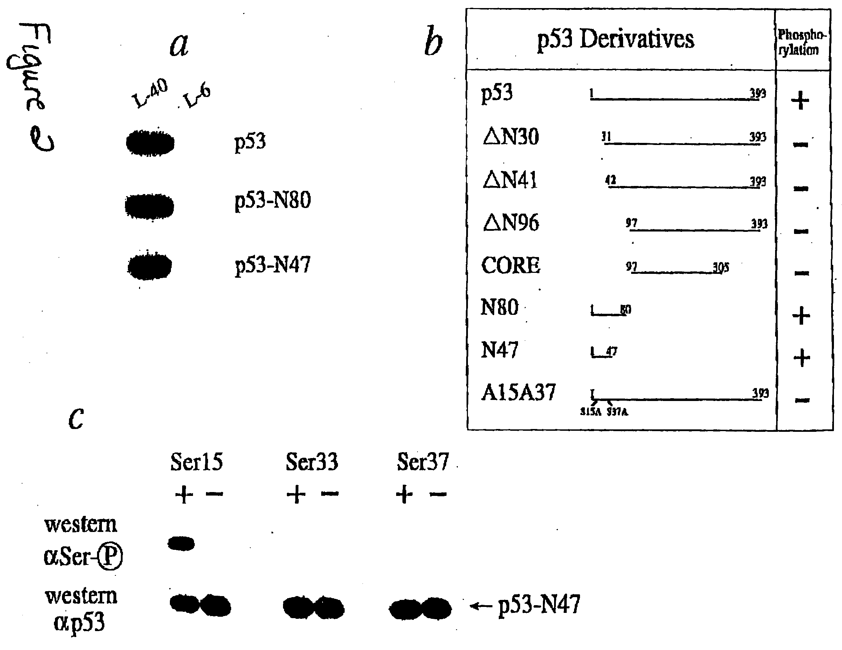

A known target of DNA damage-induced phosphorylation is the p53 protein [ref 1]. Ser15 of p53 undergoes phosphorylation in cells after DNA damage [refs 14, 15], and this response is decreased in A-T cells [ref 14]. ATM also interacts directly with p53 *in vitro* [ref 16]. Full-length recombinant p53 was phosphorylated *in vitro* by ATM immunoprecipitates (Figure 2A). Phosphorylation of p53 fragments and full-length p53 in which both Ser15 and Ser37 had been substituted by alanine residues indicated that Ser15 was the predominant, it not the only site phosphorylated by ATM (Figure 2B). Analysis with antibodies raised to p53-derived phosphopeptides containing phosphorylated serines 15, 33, or 37 [refs 14, 15, 17] (Figure 2C) confirmed that only Ser15 was phosphorylated by ATM. The effect of wortmannin on this reaction was similar to that obtained with PHAS-I as substrate (Figure 2D).

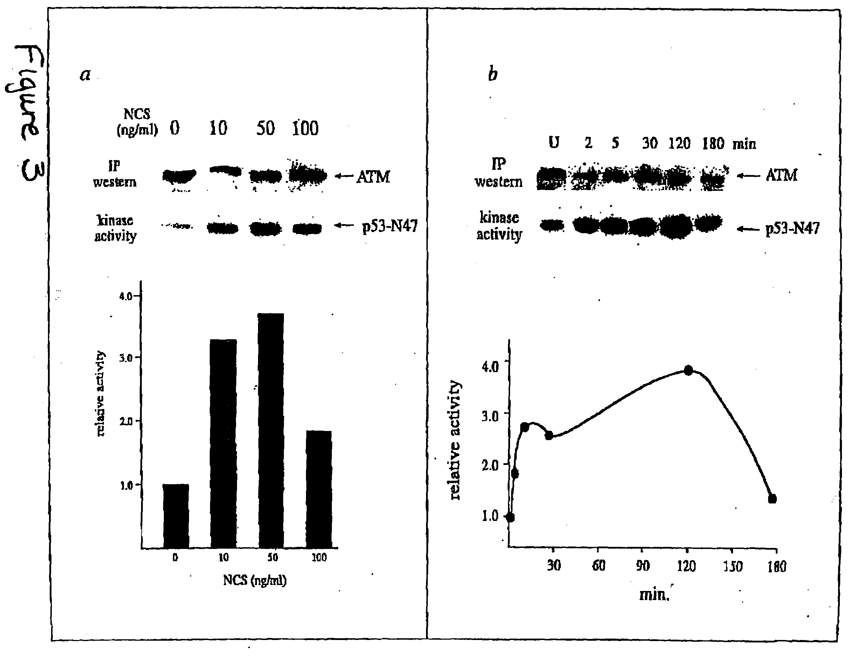

These results and the observation that damage-induced phosphorylation of Ser15 of p53 is slower in A-T cells [ref 14] suggest this residue might be a physiological target of ATM after damage caused by ionizing radiation. This amino acid is not phosphorylated in untreated cells [ref 15], so the responsible kinase must be expressed or stimulated by DNA damage. The amount of ATM remains unaltered after treatment of cells with ionizing radiation or radiomimetic drugs [ref 16,18]. However, the activity of ATM immunoprecipitated from normal lymphoblastoid cells that had been treated with various doses of the radiomimetic drug neocarzinostatin (NCS) was increased (Figure 3A). NCS belongs to a family of enediyn antibiotics that intercalate into the DNA and induce double-strand breaks by free radical attack on the deoxyribose moieties in both DNA strands. The biological effects of these compounds are similar to those of ionizing radiation [ref 19]. This enhancement was observed in five different cell lines. ATM was activated within minutes after treatment (Figure 3B). In nine experiments, the maximal enhancement of ATM's activity was 4.4±0.8 times its basal activity. Activated ATM still phosphorylated p53 only on Ser15.

Associated protein kinase activity was shown in immunoprecipitates obtained with other antibodies to ATM [ref 20]. These experiments with mutant ATM indicate that the observed activity is an intrinsic property of the ATM molecule. The biological significance of Ser15 phosphorylation on p53 is highlighted by its de novo occurrence *in vitro* after DNA damage, and its delay in A-T cells [refa 14,15]. Mutation of Ser15 leads to a reduction in the ability of p53 to arrest cell growth (21), but p53 phosphorylated at this position maintains its ability to bind to DNA [ref 15]. However, the interaction of p53 with its negative regulator, MDM2, is reduced when serines 15 and 37 are phosphorylated by DNA-dependent protein kinase and there is a reduced interaction of p53 with MDM2 after DNA damage *in vivo* [ref 15]. MDM2 can both inhibit p53-mediated transcription [ref 22] and target p53 for proteasome-mediated degradation [ref 23]. Phosphorylation of p53 at Ser15 may relieve either or both of these effects of MDM2 on p53. These finding thus point to another mechanism by which mammalian cells maintain the stability and integrity of their genome and are the basis of the present invention.

### EXAMPLE 2

Figure 1 shows the kinase activity of ATM with PHAS-I as a substrate.

Figure 1A shows the activity of ATM immunoprecipitated from the melanoma cell line G361, the normal lymphoblastoid cell line L-40, and an A-T lymphoblastoid cell line, L-6, lacking the ATM protein. Total cellular extracts (total), or ATM immunoprecipitates (IP) obtained from equal amounts of cells with the ATM132 antibody were immunoblotted with the antibody ATML2. The bottom panel shows an autoradiography of phosphorylated PHAS-I (see method herein above).

Figure 1B shows the activity of recombinant ATM stably expressed in an immortalized A-T cell line, AT22IJE-T. The cells were transfected with full-length cDNA in the episomal expression vector pEBS7, or with an empty vector. Specifically, the top panel shows the protein immunoblotting analysis of ATM immune complexes. Bottom panel: Kinase activity of ATM.

Figure 1C show the kinase activity of ATM immunoprecipitated from a normal cell line (L-40), an A-T cell line lacking the ATM protein (L-6), an A-T cell line homozygous for the truncation mutation R3047X (9RM), and an A-T cell line homozygous for the missense mutation E2904G (41RM). Since the expression of the mutant ATMs is variable, mutant cell extracts were scaled up to roughly normal ATM amounts. In all experiments, half the quantity of immune complexes was used for Western blotting analysis and the other half for the kinase reaction.

Figure 1D shows the effect of wortmannin on ATM-associated kinase activity. ATM immune complexes were incubated with various concentrations of the drug and washed with the reaction buffer before kinase activity was measured. Activity was normalized to the amount of ATM protein obtained from immunoblots. Wortmannin concentration of 80 nM was found to reduce the activity by 50%.

### EXAMPLE 3

Figure 2 shows the phosphorylation of Ser15 on p53 more specifically.

Figure 2A shows the kinase activity of ATM immunoprecipitates from L-40 and L-6 cells. Full-length p53 (top panel) and two Nh₂-terminal fragments of p53 (Figure 2B) were used as substrates. Immunoprecipitates from the A-T cell line L-6 served as a negative control.

Figure 2B shows the summary of phosphorylation of various p53 derivatives by ATM. Numbers denote the position of the terminal residues of each fragment. A15A37: Ser15 and Ser37 substituted by alanines. (P): Phosphorylation. "+" denotes phosphorylation of the substrate, "-" represents no detectable signal or signals less than 10% of that obtained with full-length p53.

Figure 2c shows the protein immunoblotting of the reaction products obtained by phosphorylating the p53-N47 fragment. Antibodies to phosphoserines 15, 33, and 37 were used in the top panel. Antibody DO1 to an epitope spanning amino acids 2 to 25 of p53 was used to detect the N47 fragment (bottom panel). "+" denotes samples taken after a phosphorylation reaction; "-" denotes the same substrate exposed to a mock reaction.

### EXAMPLE 4

Figure 3 shows the increased kinase activity of ATM immunoprecipitates from cells treated with NCS. Kinase reactions were incubated for 3 minutes. The concentration of non- radioactive ATP in the reaction mix (5 µM) was not rate-limiting.

Specifically, Figure 3A shows the ATM was immunoprecipitated from cells 20 minutes after treatment with various concentrations of NCS. Kinase activity was assayed using the p53-N47 substrate. The histogram presents quantitation of the data from the top panel (B). Phosphorylation signals were normalized against ATM amounts.

Figure 3B shows the ATM's kinase activity at various time points following treatment of the cells with NCS (50 ng/ml). U: untreated cells. The curve presents quantitation of the data from the top panel.

### EXAMPLE 5

In order to study ATM's activity, monoclonal antibodies were raised against two ATM-derived peptides, ATM132 (spanning ATM positions 819-844) and ATML2 (positions 2581-2599) and used to immunoprecipitate the protein from cultured cells. A protein kinase assay applied to immunoprecipitates obtained from a normal lymphoblastoid cell line (L-40) using the monoclonal antibody ATM132 revealed significant kinase activity when the translation repressor PHAS-I was used as a substrate (Figure 1A). This activity was significantly weaker or not detected with the commonly used substrates histone H1, casein, phosvitin and myelin basic protein. The same activity was obtained with the monoclonal antibody ATML2, but not with an antibody directed against another protein, MUC1. This activity was retained when ATM was eluted from the beads using an excess of the peptide that had been need to raise the antibody. PHAS-I phosphorylation was considerably more intense with immunoprecipitates obtained from equal cell amounts of G361, a melanoma cell line in which ATM's level is significantly higher than in lymphoblasts (Figure 1A); and it was not observed in immunoprecipitates obtained from an A-T cell line lacking the ATM protein (Figure 1A). Unlike DNA-PK, this activity was strictly dependent on manganese ions and was not enhanced by the addition of sheared double-stranded DNA.

The activity associated with recombinant ATM obtained by stable expression of ATM's cNA in an A-T cell line lacking endogenous ATM was tested. This recombinant protein is fully functional in complementing various features of the A-T cellular phenotype. Indeed, immunoprecipitates containing recombinant ATM showed kinase activity similar to that of the endogenous protein (Figure 1B).

A-T cell lines are commonly used as negative controls in studies of the ATM protein. Since A-T is usually caused by null ATM alleles, most of these cell lines completely lack this protein. While useful as initial negative controls, one cannot rule out the existence of the other protein kinases co-precipitating with ATM. The potential for ATM-associated kinase activity was therefore examined in two A-T cell lines containing structural ATM mutations that allow expression of the mutant proteins. The cell line 41RM is homozygous for a missense mutation, E2904G, replacing a highly conserved glutamic acid residue by glycine within the PI 3-kinase related domain. This region is expected to harbor the catalytic site in the PI 3-kinase related protein kinases. The cell line 9RM is homozygous for a nonsense mutation. R3047X, removing the ten carboxy-terminal residues of ATM. No significant kinase activity was detected in immunoprecipitates from either cell line, although there was a possible taint signal with the R3047X mutant (Figure 1C). The residual activity of this mutant ATM is in keeping with the clinical phenotype of the patient, who is a slightly milder variant of A-T. Taken together, these results strongly suggest that ATM has an intrinsic protein kinase activity.

PI 3-kinases are highly sensitive to inhibition by the fungal metabolite wortmannin, which covalently modifies conserved lysine residues at their catalytic sites. DNA-PK and mTOR are also sensitive to this drug. ATM kinase activity was inhibited by low concentrations of wortmannin introduced to the immune complexes for 30 minutes and washed away prior to performance of the kinase assay (Figure 1D). This result suggests an irreversible modification of ATM by wortmannin, similar to its action on the other related kinases. The degree of ATM's sensitivity to this drug (IC₅₀= 80 nM) (Figure 1D) is comparable to that of DNA-PK and of mTOR.

A known target of DNA damage-induced phosphorylation is the p53 protein. Human p53 is phosphorylated *in vitro* by DNA-PK on serine residues 15 and 37 within its transactivation domain. It was recently shown that serine-15 of p53 undergoes de novo phosphorylation *in vivo* following DNA damage, and this response is significantly retarded in A-T cells treated with ionizing radiation. Direct interaction *in vivo* between ATM and p53 has also been documented. It was therefore questioned whether p53 could be phosphorylated *in vitro* by ATM. Full-length recombinant p53 was indeed found to be a target of ATM's kinase activity (Figure 2A, top panel). To identify ATM phosphorylation sites a series of P53 derived fragments were used, as well as full-length p53 in which both serine 15 and serine 37 had been substituted by alanine residues. The results (Figures 2A,B) suggest that serine 15 was the predominant if not the only site phosphorylated by ATM on the P53 molecule. This was further supported using different antibodies raised against P53 derived phosphopeptides containing phosphorylated serines 15, 33 or 37, known to be targets of various kinases. Western blotting analysis of the phosphorylated p53-N47 fragment using the 3 antibodies (Figure 2C) indicated that of these residues only serine 15 was phosphorylated by ATM.

These results and the previous observations that damage-induced phosphorylation of serine 15 of p53 is slowed down in A-T cells suggests this residue might be a physiological target of ATM kinase following ionizing radiation damage. Since this amino acid is not constitutively phosphorylated in untreated cells, the responsible kinase is expected to be induced or stimulated by DNA damage. ATM's cellular level remains unaltered following treatment with ionizing radiation or radiomimetic drugs. It was therefore asked whether its kinase activity might be stimulated by such treatment. The activity of ATM immunoprecipitated from normal lymphoblastoid cells that had been treated with various doses of the radiomimetic drug neocarzinostatin (NCS) were tested. NCS belongs to a family of enediyn antibiotics that intercalate into the DNA and induce double-strand breaks by free radical attack on the deoxyribose moieties in both DNA strands. The biological effects of these compounds are remarkably similar to those of ionizing radiation. NCS was previously found to elicit in A-T cells the same degree of cellular hypersensitivity as ionizing radiation. ATM's kinase activity against the p53 amino-terminal fragment N47 (Figure 2B) was found to be enhanced several-told following NCS treatment, in a dose-dependent manner (Figure 3A). This enhancement was observed in five different cell lines. A time course experiment showed that ATM was activated within minutes following treatment (Figure 3B). Interestingly, a second enhancement of ATM's activity was consistently observed about 120 minutes following treatment (Figure 3B). This phenomenon reflects a second burst of DNA breaks resulting from further action of the drug, or from repair activity. In nine different experiments, the maximal enhancement of ATM's activity was 4.4 ± 0.8 fold compared to its basal activity. Activated ATM still phosphorylated p53 only on serine 15, shoving that its specificity had not been altered.

Associated protein kinase activity was previously shown in immunoprecipitates obtained with anti-ATM antibodies, but without mutant ATM controls those experiments could not establish that the observed activity was an intrinsic property of the ATM molecule. The present results indicate that ATM does indeed have intrinsic protein kinase activity, which is enhanced following DNA damage, with PHAS-I and serine 15 of p53 as *in vitro* targets. Unlike DNA-PK, ATM'S activity is not DNA-dependent, and is not directed against serine 37 of p53. However, similar to other members of the PI 3-kinase related family, ATM is sensitive to inhibition by wortmannin.

ATM phosphorylates the PHAS-I protein similar to mTOR. Following its phosphorylation *in vivo*, PHAS-I enhances the translation of transcripts containing highly structured 5' untranslated regions. mTOR indeed functions in a signaling pathway that involves PHAS-I phosphorylation following mitogenic stimulation. The biological significance of PHAS-I phosphorylation by ATM is less clear, but in view of ATM's numerous physiological roles, PHAS-I might be a plausible physiological target for its kinase activity.

At least ten residues within the amino and carboxyl termini of p53 can be phosphorylated *in vitro* by various protein kinases, but the physiological relevance of most of these phosphorylations is unclear. The biological significance of serine 15 phosphorylation is highlighted by its de novo occurrence *in vivo* following DNA damage, and retardation of the process in A-T cells. In addition, Fiscella et al. showed that mutation of serine 15 leads to a reduction in the ability of p53 to arrest cell growth. p53 phosphorylated at this position maintains its ability to bind to DNA. However, the interaction of p53 with its negative regulator, MDM2, is reduced when serines 15 and 37 are phosphorylated by DNA-PK and there is a reduced interaction of p53 with MDM2 after DNA damage *in vivo*. MDM2 can both inhibit p53 mediated transcription and target p53 for proteasome-mediated degradation. Phosphorylation of p53 at serine 15 relieves either or both of the effects of MDM2 on p53. Interestingly, while ATM deficient cells exhibit delayed accumulation of p53 following radiation damage, cells deficient in DNA-PK show normal p53 response and normal activation of the G1/S checkpoint. These observations show that, despite the redundancy of kinases that phosphorylate serine 15 of p53, ATM's activity is perhaps more central in p53's response to DNA breaks.

More than one modification of the p53 molecule is needed for its full activation by DNA damage. Siliciano et al. noted in irradiated cells at least one phosphorylation event at the amino terminus of p53 in addition to serine 15 phosphorylation. Recently, Waterman et al. observed an ATM-dependent dephosphorylation of serine 376 of p53 following radiation damage. This additional ATM-dependent pathway, which probably involves other proteins operating between ATM and p53, contributes to the enhancement of p53 transactivation capacity. Thus, ATM regulates two different properties of the same downstream effector via different pathways. Since ATM is involved in additional signaling pathways, related or unrelated to DNA damage, its kinase activity is directed against numerous cellular targets, which changes according to the initial signal or the physiological state of the cell.

Finding that treatment of cells with a NA breaking agent enhances ATM's activity shows that the enhanced rather than the basal activity of ATM is responding to the damage. The activation is due either to a structural modification of the ATM molecule, or to its increased association with a protein cofactor. This novel cellular response to DNA strand breaks uncovers another mechanism by which mammalian cells maintain the stability and integrity of their genome.

In summary, based on Examples 1-5, there is described a method for modulating the ATM protein by effecting DNA strand breaks. As described in the examples, the activity of the ATM protein can be modulating by effecting DNA strand breaks. Also, described are the antibodies which are directed against the ATM protein.

Throughout this application, various publications, including United States patents, are referenced by author and year or by number and patents by number. Full citations for the publications are listed below. The disclosures of these publications and patents in their entireties are hereby incorporated by reference into this application in order to more fully describe the state of the art to which this invention pertains.

The invention has been described in an illustrative manner, and it is to be understood that the terminology which has been used is intended to be in the nature of words of description rather than of limitation.

Obviously, many modifications and variations of the present invention are possible in light of the above teachings. It is, therefore, to be understood that within the scope of the described invention, the invention may be practiced otherwise than as specifically described.

### REFERENCES

Gilboa, E, Eglitis, MA, Kantoff, PW, Anderson, WF: Transfer and expression of cloned genes using retroviral vectors. BioTechniques 4(6):504-512, 1986.
Cregg JM, Vedvick TS, Raschke WC: Recent Advances in the Expression of Foreign Genes in *Pichia pastoris*, Bio/Technology 11:905-910, 1993
Culver, 1998. Site-Directed recombination for repair of mutations in the human ADA gene. (Abstract) Antisense DNA & RNA based therapeutics, February, 1998, Coronado, CA.
Huston et al, 1991 "Protein engineering of single-chain Fv analogs and fusion proteins" in Methods in Enzymology (JJ Langone, ed.; Academic Press, New York, NY) 203:46-88.
Johnson and Bird, 1991 "Construction of single-chain Fvb derivatives of monoclonal antibodies and their production in *Escherichia coli* in Methods in Enzymology (JJ Langone, ed.; Academic Press, New York, NY) 203:88-99.
Mernaugh and Mernaugh, 1995 "An overview of phage-displayed recombinant antibodies" in Molecular Methods In Plant Pathology (RP Singh and US Singh, eds.; CRC Press Inc., Boca Raton, FL) pp. 359-365.
Agrawal, 1996. Antisense oligonucleotides: towards clinical trials, TIBTECH, 14:376.
Akhter et al, 1991. Interactions of antisense DNA oligonucleotide analogs with phospholipid membranes (liposomes). Nuc. Res. 19:5551-5559.
Blaesse, 1997. Gene Therapy for Cancer. Scientific American 276(6):111-115.
Calabretta, et al, 1996. Antisense strategies in the treatment of leukemias. Semin. Oncol. 23:78.
Crooke, 1995. Progress in antisense therapeutics, Hematol. Pathol. 2:59.
Eckstein 1985. Nucleoside Phosphorothioates. Ann. Rev. Biochem. 54:367-402.
Felgner, 1997. Nonviral Strategeies for Gene Therapy. Scinetific American. June, 1997, pgs 102-106.
Gewirtz, 1993. Oligodeoxynucleotide-based therapeutics for human leukemias, Stem Cells Dayt. 11:96.
Galileo et al., 1991. J. Cell. Biol., 112:1285.
Hanania, et al 1995. Recent advances in the application of gene therapy to human disease. Am. J. Med. 99:537.
Iyer et al. 1990. J. Org. Chem. 55:4693-4699.
Lefebvre-d'Hellencourt et al, 1995. Immunomodulation by cytokine antisense oligonucleotides. Eur. Cytokine Netw. 6:7.
Lev-Lehman et al., 1997. Antisense Oligomers *in vitro* and *in vivo.* In Antisense Therapeutics, A. Cohen and S. Smicek, eds (Plenum Press, New York)
Loke et al, 1989. Characterization of oligonucleotide transport into living cells. PNAS USA 86:3474.
Morrison, 1991. Suppression of basic fibroblast growth factor expression by antisense oligonucleotides inhibits the growth of transformed human astrocytes. J. Biol. Chem. 266:728.
Radhakrishnan et al., 1990. The automated synthesis of sulfur-containing oligodeoxyribonucleotides using 3*H*-1,2-Benzodithiol-3-0ne 1,1 Dioxide as a sulfur-transfer reagent. J. Org. Chem. 55:4693-4699.
Rosolen et al., 1990. Cancer Res. 50:6316.
Scanlon et al., 1995. Oligonucleotides-mediated modulation of mammalian gene expression. FASEB J. 9:1288.
Shaw et al., 1991. Modified deoxyoligonucleotides stable to exonuclease degradation in serum. Nucleic Acids Res. 19:747-750.
Spitzer and Eckstein 1988. Inhibition of deoxynucleases by phosphorothioate groups in oligodeoxyribonucleotides. Nucleic Acids Res. 18:11691-11704.
Uhlmann and Peyman, 1990. Antisense Oligonucleotides: A New Therapeutic Principle. Chem Rev 90(4):543-584.
Wagner et al., 1996. Potent and selective inhibition of gene expression by an antisense heptanucleotide. Nature Biotechnology 14:840-844.
Wagner, 1994. Gene inhibition using antisense oligodeoxynucleotides. Nature 372:333.
Whitesell et al., 1991. Episome-generated N-*myc* antisense RNA restricts the differentiation potential of primitive neuroectodermal cell lines. Mol. Cell. Biol. 11:1360.
Yakubov et al, 1989. PNAS USA 86:6454.
Wright & Anazodo, 1995. Antisense Molecules and Their Potential For The Treatment Of Cancer and AIDS. Cancer J. 8:185-189.
Woolf et al., 1990. The stability, toxicity and effectiveness of unmodified and phosphorothioate antisense oligodeoxynucleotides in *Xenopus* oocytes and embryos. Nucleic Acids Res. 18:1763-1769.
Burke and Olson, 1991. "Preparation of Clone Libraries in Yeast Artificial-Chromosome Vectors" in Methods in Enzymology, Vol. 194, "Guide to Yeast Genetics and Molecular Biology", eds. C. Guthrie and G. Fink, Academic Press, Inc., Chap. 17, pp. 251-270.
Capecchi, 1989. "Altering the genome by homologous recombination" Science 244:1288-1292.
Davies et al., 1992. "Targeted alterations in yeast artificial chromosomes for inter-species gene transfer", Nucleic Acids Research, 20(11):2693-2698.
Dickinson et al., 1993. "High frequency gene targeting using insertional vectors", Human Molecular Genetics, 2(8):1299-1302.
Duff and Lincoln, 1995. "Insertion of a pathogenic mutation into a yeast artificial chromosome containing the human APP gene and expression in ES cells", Research Advances in Alzheimer's Disease and Related Disorders.
Herskowitz (1987). Functional Inactivation of Genes By Dominant Negative Mutations. Nature 329(6136):219-222.
Holzmayer et al., (1932). Isolation of Dominant Negaive Mutants and Inhibitory Antisense RNA Sequences by Expression Selection of Random DNA Fragments. Nucleic Acids Res 20(4):711-717.
Lamb et al., 1993. "Introduction and expression of the 400 kilobase *precursor amyloid protein* gene in transgenic mice", Nature Genetics, 5:22-29.
Lavitrano at al, 1989. Cell 57;717-723
Lo, 1983. Mol. Cell. Biol. 3:1803-1814
Mansour, 1990. Gene targeting in murine embryonic stem cells: Introduction of specific alterations into the mammalian genome. GATA 7(8):219-227.
Rothstein, 1991. "Targeting, disruption, replacement, and allele rescue: integrative DNA transformation in yeast" in Methods in Enzymology, Vol. 194, "Guide to Yeast Genetics and Molecular Biology", eds. C. Guthrie and G. Fink, Academic Press, Inc., Chap. 19, pp. 281-301.
Schedl et al., 1993. "A yeast artificial chromosome covering the tyrosinase gene confers copy number-dependent expression in transgenic mice", Nature, 362:258-261.
Thompson at al, 1989. Cell 56:313-321 Van der Putten at al, 1985. PNAS USA 82:6148-6152
   Additional references referred to in text by number:
   1. A. J. Levine, *Cell* **88,** 323 (1997).
   2. M. Lavin, Y. Shiloh, *Ann. Rev. Immunol.* **15**, 177 (1997); Y. Shiloh, *Ann. Rev. Genet.* **31**, 635 (1997); and R. A. Gatti, In *The Genetic Basis of Human Cancer*, B.Vogelstein, K. W. Kinzler, Eds. (McGraw-Hill, N.Y., 1998), pp. 275-300; and C. Barlow et al., *Cell* **86**, 159 (1996); and Y. Xu and D. Baltimore, *Genes Dev*. **10**, 2401 (1996); and S. E. Morgan, M. B. Kastan, *Adv. Cancer Res*. **71**, 1 (1997).
   3. K. Savitsky et al., Science **268**, 1749 (1995); and K. Savitsky et al., *Hum. Mol. Genet*. **4**, 2025 (1995).
   4. M. F. Hoekstra, *Curr*. *Opin*. *Genet*. *Dev*. **7**, 170 (1997).
   5. K. O. Hartley et al., *Cell* **82**, 849 (1995); and E. J. Brown et al., *Nature* **377**, 441 (1995); and G. I. Brunn et al., *EMBO J*. **15**, 5256 (1996); and G.J. Brunn et al., Science **277**, 99 (1997).
   8. Y. Ziv et al., *Oncogene* **15**, 159 (1997).
   9. T-. A. Lin et al., *Science* **266**, 653 (1994).
   11. S. Gilad et al., *Hum. Mol. Genet*. **5**, 433 (1996).
   12. S. Gilad et al., *Am. J. Hum. Genet*. **62**, 551 (1998).
   13. M. T. Ui, T. Okada, K. Hazeki, O. Hazeki, *Trends Biochem*. *Sci*. **20**, 303 (1995); and M. P. Wymann et al., *Mol*. *Cell Biol*. **16**, 1722 (1996).
   14. J. D. Siliciano et al., *Genes Dev*. **11**, 3471 (1997).
   15. S.- Y. Shieh, M. Ikeda, Y. Taya, C. Prives, *Cell* **91**, 325 (1997).
   16. D. Watters et al., *Oncogene* **14**, 1911 (1997).
   17. M. Kitagawa et al., *EMBO J*. **15**, 7060 (1996); and L. J. Ko et al., *Mol. Cell. Biol*. **17**, 7220 (1997).
   18. N. D. Lakin et al., *Oncogene* **13**, 2707 (1996); K. D. Brown et al., Proc. Natl. Acad. Sci. USA **94**, 1840 (1997).
   19. I. H. Goldberg, L. S. Kappen, In *Enediyne Antibiotics as Antitumor Agents*, D.B. Borders, T. W. Doyle. Eds. (Marcel Dekker Press, N.Y., 1995) pp. 327-362;
   20. K. S. Keegan et al., *Genes De*v. **10**, 2423 (1996); and M. Jung et al., *Cancer Res*. **57**, 24 (1997).
   21. M. Fiscella et al., *Oncogene* **8**, 1519 (1993).
   22. J. Momand, G. P. Zambetti, D. C. Olson, D. george, A. J. Levine, *Cell* **69**, 1237 (1992); and J. D. Oliner et al., *Nature* **362**, 857 (1993).
   23. Y. Haupt, R. Maya, A. Kazaz, M. Oren, *Nature* **387**, 286 (1997); and M. H. G. Kubbutat, S. N. jones, K. H. Vousden, *Nature* **387**, 299 (1997).
L.H. Hartwell, B.B. Kastan, Science 266, 1821 (1994); A.G. Paulovich, D.P. Toczyski, L.H. Hartwell, Cell 88, 315 (1997); A.R. Lehmann, BioEssays 20, 146 (1998); J.A. Nickoloff, M.F. Hoekstra, DNA Damage and Repair (Humana Press, 1998) Vols. 1,2.
T.M. Gottlieb, M. Oren, Biochim. Biophys. Acta 1287, 77 (1996); L.J. Ko, D. Prives, Genes Dev. 10, 1054 (1996); A.J. Levine, Cell 88, 323 (1997).
M. Lavin, Y. Shiloh, Ann. Rev. Immuno. 15, 177 (1997); Y. Shiloh, Ann. Rev. Genet. 31,635 (1997); R.A. Gatti, In the Genetic Basis of Human Cancer, B. Vogelstein, K.W. Kinzler, Eds. (McGraw-Hill, N.Y., 1998) pp. 275-300; c. Barlow et al., Cell 86, 159 (1996); Y. Xu et al.., Genes Dev. 10, 2401.
M.B. Kastan et al., Cell 71, 589 (1992); C.E. Canman et al., Cancer Res. 54, 5054 (1994); K.K. Khanna et al., Oncogene 11, 609 (1995); S.E. Morgan, M.B. Kastan, Adv. Cancer Res. 71, (1997).
K. Savitsky et al., Science 268, 1749 (1995); K. Savitsky et al., Hum. Mol. Genet. 4, 2025 (1995).
V.A. Zakian, cell 82, 685 (1995); C.T. Keith, S.L. Schreiber, Science 270, 51 (1995); T. Hunter, Cell 83, 1 (1996); R.T. Abraham Curr. Opin. Immunol. 8, 412 (1996); M.F. Hoekstra, cirr. Opin. Genet. Dev. 7, 170 (1997).
K.O. Hartley et al., Cell 82, 849 (1995); P.A. Jeggo, Mut. Res. 384, 1 (1997).
E.J. Brown et al., Nature 377, 441 (1995); G.I. Brunn et al., Embro J. 15, 5256 (1996); G.J. Brunn et al., Science 277, 99 (1997); P.E. Burnett et al., Proc. Natl. Acad. Sci. USA 95, 1432 (1998); G. Thomas, M.N. Hall, Curr. Opin. Cell Biol. 9, 782 (1997).
L. Moyal et al., in preparation.
Y.Ziv et al., Oncogene 15, 159 (1997).
A. Pause at al., Nature 371, 762 (1994); C. Hu, S. Pang, X. Kong, M. Velleca, J.C. Lawrence Jr., Proc. Natl. Acad. Sci. USA 91, 3730 (1994); T.A. Lin et al., Science 266, 653 (1994).
I. Keydar et al., Proc. natl. Acad. Sci. USA, 96, 1362 (1989).
P.T. Peebles, T. Trisch, A.G. Papageorge, Pediatr. Res. 12, 485 (1978).
S. Gilad et al., Hum. Mol. Genet. 5, 433 (1996).
S. Gilad et al., Am. J. Hum. Genet. 62, 551 (1998).
A. Arcaro, M.P. Wyman, Biochem. J. 296, 297 (1993); G. Powis et al., Cancer Res. 54, 2419 (1994); M. T. Ui, T. Okada, K. Hazeki, O. Hazeki, Trends Biochem. Sci. 20,303 (1995); M.P. Wymann et al., Mol. Cell Biol. 16, 21733 (1996).
S.P. Lees-Miller et al., Mol. Cell Biol. 12, 5041 (1992).
J.D. Siliciano et al., Genes. Dev. 11, 3471 (1997).
S.Y. Shieh, M. Ikeda, Y. Taya, C. Prives, Cell 91, 325 (1997).
D. Watters at al., Oncogene 14, 1911 (1997).
M. Kitagawa at al., Embo J. 15, 7060 (1996); L.J. Ko et al., Mol. Cell Biol. 17, 7220 (1997); S. Y. Shieh, Y. Taya, C. Prives, in preparation.
B. Vojtesek, J. Bartek, C.A. Midgley, D.P. Lane, J. Immunol. Methods 151, 237 (1992); Y. Lagros, C. Laton, T. Soussi, Oncogene 9, 2071 (1994).
N.D. Lakin et al., Oncogene 13, 2707 (1996); K.D. Brown et al., Proc. Natl. Acad. Sci. USA 94, 1840 (1997).
I.H. Goldberg, L.S. Kapen, In Enediyne Antiboitics as Antitumor Agents. D. B. Borders, T.W. Doyle, Eds. (Marcel Dekker Press, N.Y. 1995) pp. 327-362; L.F. Povirk, Mutat. Res. 355, 71 (1996); Z. Xi, I.H. Goldberg, In Comprehensive Natural Product Chemistry, D.H.R. Barton, K. Nakanishi, O. Meth-Cohn, Eds. (Elsevier, Oxford 1998) vol. 7 (in press).
Y. Shiloh, E. Tabor, Y. Becker, Cancer Res. 42, 2247 (1982).
K.S. Keegan et al., Genes Dev. 10, 2423 (1996); M. Jung et al., Cancer Res. 57, 24 (1997).
G.J. Milczarek, J. Martinez, G.T. Bowden, Life Sci. 60, 1 (1997); D. Meek, Cell signal. 10, 159 (1998).
M. Fiscella et al., Oncogene 8, 1519 (1993).
J. Momand, G.P. Zambetti D.C. Olso, D. George, A.J. Levine, Cell 69, 1237 (1992); J.D. Oliner et al., Nature 362, 857 (1993).
Y. Haupt, R. Maya, A. Kazaz, M. Oren, Nature 387, 286 (1997); M.H.G. Kubbutat, S.N. Jones, K.H. Vousden, Nature 387, 299 (1997).
M.A. Bogue et al., Genes Dev. 10, 553 (1996); C.J. Guidos et al., Genes Dev. 10, 2038 (1996); K.E. Gurley, G.J. Kemp, Carcinogenesis 17, 2537 (1996); L. Huang, K.C. Clarkin and G.M. Wahl, Cancer Res., 56, 2940 (1996); W.K. Rathmell et a., Cancer Res. 57, 68 (1997).
M.J.F. Waterman, E.S. Stravridi, J.L.F. Wateran, T.D. Halozonetis, Nature Genet. (in press).

## Claims

1. Antibodies directed against the ATM protein or peptide derivatives of the protein.

2. The antibodies according to claim 1, wherein said antibodies are further characterized as being able to recognize endogenous, recombinant and *in vitro* translated ATM immunoprecipitated by other anti-ATM antibodies.

3. The antibodies according to claim 1, wherein said antibodies are further characterized as being able to recognize a 379 kD protein which reappears upon ectopic expression of ATM in cells.

4. A diagnostic tool for determining the presence of ATM comprising detection means for detecting ATM protein levels and quantification means for analyzing the ATM protein activity.

5. A kit for detecting the presence of ATM comprising detection means for detecting ATM protein levels and quantification means for analyzing the ATM protein activity.

6. A method of studying ATM activity by;
raising antibodies against ATM-derived peptides;
immunoprecipitating ATM proteins from the cultured cells; and
assaying the immunoprecipitates.

7. The method according to claim 6, further including the step of observing the intrinsic protein kinase activity of ATM, wherein endogenous or recombinant ATM has wortmannin-sensitive protein kinase activity and AT⁺ mutations do not have wortmannin-sensitive protein kinase activity.
